(19) Europäisches Patentamt
European Patent Office
Office européen des brevets

(11) **EP 1 046 909 B1**

(12) **FASCICULE DE BREVET EUROPEEN**

(45) Date de publication et mention
de la délivrance du brevet:
**17.11.2010  Bulletin 2010/46**

(51) Int Cl.:
***G01N 33/28*** (2006.01)

(21) Numéro de dépôt: **00400903.1**

(22) Date de dépôt: **30.03.2000**

(54) **Méthode et dispositif pour la détermination du coefficient de Joule-Thomson d'un fluide**

Verfahren und Vorrichtung zur Messung des Joule-Thomson Koeffizienten eines Fluids

Method and apparatus for determining the Joule-Thomson coefficient of a fluid

(84) Etats contractants désignés:
**DK GB NL**

(30) Priorité: **23.04.1999  FR 9905227**

(43) Date de publication de la demande:
**25.10.2000  Bulletin 2000/43**

(73) Titulaire: **Institut Français du Pétrole
92852 Rueil-Malmaison Cedex (FR)**

(72) Inventeurs:
• **Mougin, Pascal
92500 Rueil Malmaison (FR)**
• **Peumery, Roxane
92500 Rueil Malmaison (FR)**
• **Moracchini, Gérard
95580 Andilly (FR)**
• **Sanchez, José
95270 Viarmes (FR)**

(56) Documents cités:
**EP-A- 0 070 188**

• **DATABASE WPI Derwent Publications Ltd., London, GB; AN 1993-108692 XP002128515 PETROV E. K.: "gas joule-thomson effect meter" (MOSC POWER INT), 1993 & SU 1 732 191 A (PETROV E. K) 7 mai 1992 (1992-05-07)**
• **DATABASE WPI , 1981 Derwent Publications Ltd., London, GB; AN 1981-63604D XP002128516 FILIPPOV A.: "joule-thomson coefficient determination for fluids" (BASHKIR UNIV.) & SU 777 557 A (FILIPPOV) 7 novembre 1980 (1980-11-07)**
• **FILIPPOV A.I. ET AL: 'CALCULATION OF THE THERMAL FIELD OF THE THROTTLE ELEMENT OF APPARATUS FOR STUDYING THE JOULE-THOMSON EFFECT' JOURNAL OF ENGINEERING PHYSICS, XX, XX LNKD- DOI: 10.1007/BF00861881 vol. 38, no. 2, 01 Janvier 1980, pages 203 - 207, XP000870228 ISSN: 0022-0841**
• **BIER K. ET AL: 'Flow apparatus for measuring the heat capacity and the Joule-Thomson coefficient of gases' JOURNAL OF CHEMICAL THERMODYNAMICS, ACADEMIC PRESS, LONDON, GB LNKD- DOI:10.1016/0021-9614(74)90065-2 vol. 6, no. 11, 01 Novembre 1974, pages 1027 - 1037, XP023945778 ISSN: 0021-9614**

## Description

La présente invention concerne une méthode et un dispositif pour la mesure de coefficients de Joule-Thomson de fluides.

Le coefficient de Joule-Thomson $\mu$ mesure la variation de la température d'un fluide soumis à une chute de pression en situation isenthalpique.

$$\mu = \left( \frac{\partial T}{\partial P} \right)_H$$

Cette condition d'isenthalpie est précisément celle que l'on rencontre lors de la détente d'un fluide dans une vanne ou dans une conduite à condition de pouvoir négliger les dissipations d'énergie vers l'extérieur.

La mesure précise du coefficient de Joule-Thomson trouve des applications dans de nombreux domaines où l'acheminement de fluides dans des conduits provoque des changements d'état nuisibles à leur bonne circulation, et notamment dans le domaine de l'exploitation de gisements d'hydrocarbures à haute pression - haute température (HP - HT). Par cette mesure, on peut déterminer le « profil thermique » dans tous les organes dissipatifs d'énergie.

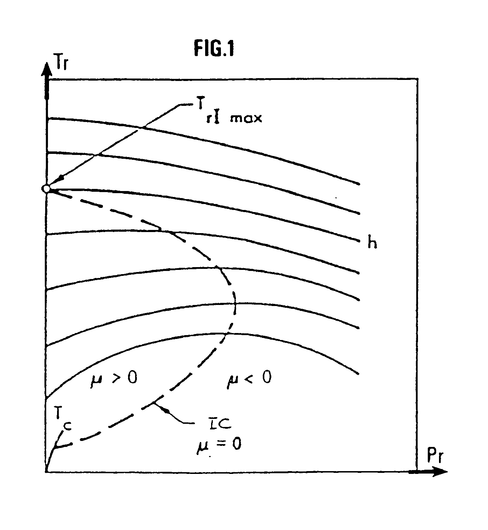

Selon les conditions de température et de pression, le coefficient de Joule-Thomson $\mu$ peut être positif ou négatif, comme le montre la Fig. 1. Dans le cas d'un coefficient positif ($\mu > 0$), le gaz se refroidit lors d'une détente tandis qu'un coefficient négatif ($\mu < 0$) conduit à un réchauffement par détente. Le domaine positif du coefficient est séparé du domaine négatif par la courbe d'inversion IC. On constate que dans les conditions HP - HT régnant dans un puits à grande profondeur, le coefficient $\mu$ est négatif : par détente, le fluide s'échauffe. C'est ce que l'on observe à l'heure actuelle dans les puits de production des gisements à profondeur relativement grande et notamment dans certains puits de la Mer du Nord produisant des gaz à condensat où les conditions HP - HT provoquent une inversion du coefficient de Joule-Thomson.

Le signe et la valeur du coefficient de Joule-Thomson sont donc importants pour le dimensionnement d'un puits de production puisqu'il conditionne le profil thermique des installations de production. Dans le cas d'un coefficient négatif, il est impératif de connaître l'échauffement réalisé par détente : le choix des matériaux de construction en dépend. Ce coefficient peut également être utilisé pour le dimensionnement des conduites de gaz pour les mêmes raisons. Connaître le signe et la valeur de ce coefficient est également nécessaire pour évaluer les risques de formation d'hydrates ou de paraffines en cas d'une baisse de la température par détente, de façon à bien choisir la technique appropriée permettant de combattre la formation de dépôts dans les conduites.

## Etat de la technique

Il existe dans la littérature de nombreuses références montrant comment on calcule la courbe IC(P, T) d'inversion du coefficient de Joule-Thomson à l'aide d'équations d'état classiquement utilisées dans l'industrie pétrolière. On peut se référer notamment à :

- Kortekaas W.G., et al ; Joule-Thomson Expansion of High Pressure-High-Temperature Gas Condensates, in Fluid Phase Equilibria, 139, 1997, p. 207-218.

Cependant, cette approche est difficilement exploitable dans la pratique faute de disposer des données expérimentales nécessaires qui sont rarement divulguées. De plus, la mesure du coefficient de Joule-Thomson $\mu$ est délicate puisque l'on doit apprécier des valeurs absolues très faibles de l'ordre de quelques dizièmes de °C/bar (quelques °C/Mpa). Une bonne determination de la courbe d'inversion IC demande une grande précision puisque l'écart de température observable est très voisin de 0.

Il existe différents types de dispositifs expérimentaux permettant de déterminer le coefficient de Joule-Thomson par la mesure de la variation de température d'un fluide traversant un organe.

Suivant un premier mode de mise en oeuvre, cet organe est constitué d'un milieu poreux provoquant une perte de charge qui est fonction de sa perméabilité et du débit de fluide. Un tel dispositif présente des inconvénients liés au fait que l'effet Joule-Thomson est dispersé dans tout le volume poreux qui est difficile à isoler thermiquement, et possède une certaine inertie thermique, ce qui demande une grande quantité de fluide pour atteindre l'état d'équilibre thermique lors de la mesure.

Suivant un deuxième mode de mise en oeuvre, l'organe provoquant une perte de charge est constitué d'une vanne. C'est une solution avantageuse car ici il est facile de faire varier la différence de pression entre l'entrée et la sortie du dispositif. De plus, l'effet Joule-Thomson est assez bien localisé, avec cependant l'inconvénient que l'ensemble de ses pièces telles que le siège, le pointeau, etc., forme une masse thermique qui produit des fuites thermiques difficiles à éviter.

L'assurance d'une bonne isolation thermique et une bonne localisation de l'effet Joule-Thomson sont les points clés d'une bonne mesure. Ce sont les qualités du dispositif selon l'invention.

## Définition de l'invention

La méthode selon l'invention permet de déterminer le coefficient de Joule-Thomson d'un fluide par combinaison de mesures des variations concomitantes de pression et de température subies par un fluide en circulation. Elle comporte une injection de ce fluide à une température déterminée dans un tube fin dans lequel est placée une sonde de mesure de température provoquant une perte de charge pour le fluide, une mesure, au moyen de cette sonde, de la variation de la température du fluide par rapport à sa température d'injection, une mesure de la perte de charge subie par le fluide et une détermination du coefficient de Joule-Thomson par combinaison des mesures de perte de charge et de variation de température du fluide. De préférence, on confine thermiquement le tube fin pour éviter les déperditions de chaleur.

Le dispositif selon l'invention permet une détermination du coefficient de Joule-Thomson d'un fluide, sous pression. Il comporte un tube fin, des moyens d'injection du fluide dans le tube fin à une température déterminée, une première sonde de mesure dans ce tube fin créant une perte de charge et adaptée à mesurer la variation de la température du fluide ayant subi cette perte de charge, des capteurs de pression en amont et en aval de ce tube pour mesurer la perte de charge, une deuxième sonde de mesure de la température du fluide injecté, et un moyen de calcul pour déterminer le coefficient de Joule-Thomson du fluide à partir de cette perte de charge et de cette variation de température.

De préférence, le dispositif comporte des moyens de confinement pour isoler thermiquement le tube fin, comportant par exemple un tube de confinement contenant le tube fin, ce tube de confinement étant pourvu d'embouts à ses deux extrémités opposées délimitant avec lui une enceinte étanche, des canaux dans ces deux embouts pour mettre l'intérieur du tube fin en communication avec les moyens d'injection de fluide, l'intérieur du tube de confinement en communication avec des moyens pour y faire le vide, et pour mettre le tube fin en communication avec les moyens de pression.

Suivant un mode de réalisation, les moyens de confinement comportent en outre, un tube intermédiaire entre le tube fin et le tube de confinement. L'un au moins des tubes autour du tube fin est pourvu d'un revêtement réfléchissant sur sa paroi intérieure de manière à éviter les déperditions d'énergie par rayonnement.

De préférence, le dispositif comporte une unité de conditionnement du fluide en température avant son injection.

Les sondes de température sont de préférence des thermocouples identiques montés en opposition de manière à détecter de faibles variations de température.

L'utilisation d'une vanne de régulation du débit sortant permet de bien vérifier que la mesure du coefficient de Joule-Thomson est bien indépendante du débit.

La mesure très bien localisée à l'extrémité de la zone de perte de charge dans le tube fin, combinée de préférence avec un bon confinement du tube fin évitant sensiblement toute perte thermique dans la zone de mesure, garantit une très bonne précision.

## Figures

D'autres caractéristiques et avantages du dispositif selon l'invention, apparaîtront à la lecture de la description ci-après d'un exemple non limitatif de réalisation du dispositif, en se référant aux dessins annexés où :

- la Fig. 1 montre un réseau de courbes de variation, à enthalpie constante, de la température Tr d'un fluide en fonction de sa pression Pr ;

- la Fig.2 montre une vue en coupe schématique du dispositif ;

- les Fig.2A, 2B sont des vues en coupe transversales respectivement des embouts 2A et 2B ;

- les Fig.3 à 5 montrent les mesures expérimentales des variations du coefficient de Joule-Thomson J de l'azote en fonction du débit F respectivement pour des pressions de 5, 10 et 15 Mpa, obtenues par la mise en oeuvre du dispositif et, à titre de comparaison, les valeurs J1 à J3 que l'on obtient par le calcul à partir de l'équation d'état spécifique de ce gaz respectivement pour les mêmes pressions ; et

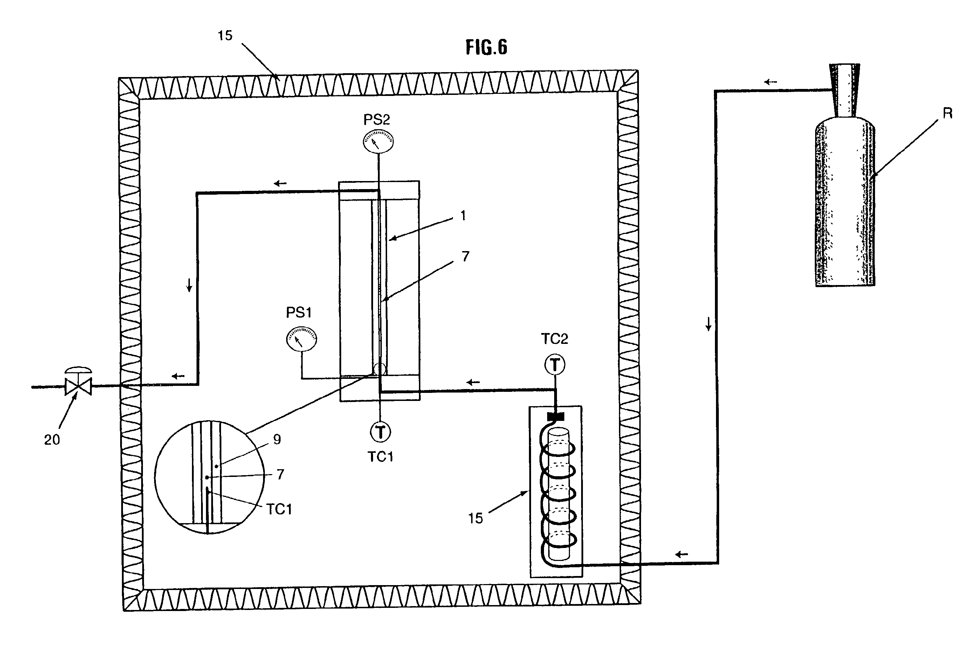

- la Fig.6 montre schématiquement une vue d'ensemble du dispositif dans son enceinte thermostatée.

## Description détaillée

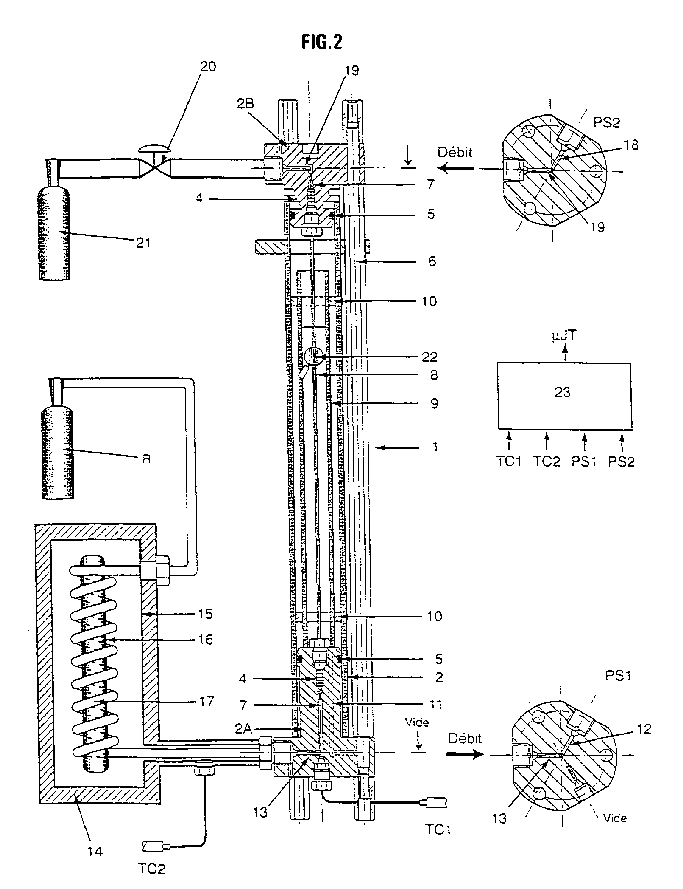

Le dispositif comporte (Fig.2) une unité de mesure 1. Elle comprend un tube extérieur 2 de préférence revêtu intérieurement d'une couche métallique réfléchissante. Le tube extérieur est associé à deux embouts 2A, 2B, que l'on désignera ci-après respectivement par embout amont et embout aval. Chacun d'eux comprend un prolongement cyclindrique 4 de diamètre adapté au diamètre intérieur du tube 2, pourvu extérieurement d'un joint torique d'étanchéité 5. Les deux embouts 2A, 2B sont réunis par une entretoise 6 associée à des moyens de fixation non représentés. Chacun des embouts comporte un canal axial 7.

Les canaux axiaux respectifs des deux embouts 2A, 2B sont réunis par un tube métallique fin 8. Un tube intermédiaire 9 est disposé autour du tube fin 8. Il est positionné par rapport au tube extérieur 2, au moyen de bagues en matière plastique 10. Ce tube intermédiaire 9 est réalisé par exemple en verre et il peut être revêtu intérieurement d'une couche métallique réfléchissante tel qu'un revêtement argenté. Un canal 11 est ménagé au travers de l'embout amont 2A. Il débouche dans le tube intermédiaire 9 et communique extérieurement avec une pompe à vide P. L'action combinée du revêtement argenté sur la paroi interne du tube extérieur 2 et éventuellement du tube intermédiaire 9, et du vide régnant en opération à l'intérieur des tubes 2 et 9, permet de limiter au mieux les pertes calorifiques par conduction et rayonnement.

Le canal axial 7 dans l'embout amont 2A, communique par un premier canal radial 12, avec un premier capteur de pression PS1, et par un deuxième canal radial 13, avec des moyens d'injection de fluide R par l'intermédiaire d'une unité de stabilisation en température 14. Cette unité comporte par exemple une enceinte thermostatée 15 connectée avec l'unité de mesure 1. Dans cette enceinte 15, est placé un échangeur de chaleur comprenant par exemple une bobine de cuivre 16 sur laquelle est enroulé étroitement un tube fin 17 (un capillaire par exemple) d'amenée du fluide à étudier. Selon les cas, la bobine de cuivre 16 peut rester vide et communiquer passivement au tube 17 la température de l'enceinte, au tube d'amenée du gaz, ou bien elle peut être raccordée à un circuit primaire (non représenté) où circule un fluide chauffé.

Le canal axial 7 dans l'embout aval 2B communique par un premier canal radial 18, avec un capteur de pression PS2, et par un deuxième canal 19 soit directement avec l'extérieur (échappement libre), soit, si le fluide mesuré doit être récupéré pour d'autres mesures éventuelles (par exemple du fluide de gisement disponible généralement en quantité limitée), avec une chambre de récupération du fluide 21, par l'intermédiaire d'une vanne 20 de régulation précise de débit, de type à vis micrométrique par exemple.

Par le canal axial 7 au travers de l'embout amont 2A, on introduit dans le tube fin 8 sur une partie de sa longueur, une première sonde de température, en l'occurrence un premier thermocouple TC1. Un deuxième thermocouple TC2 est placé dans la zone de raccordement de l'unité de stabilisation de température 14 avec l'unité de mesure 1. Les deux thermocouples sont identiques. Ils sont issus des mêmes bobinages de fil, et montés en opposition afin de mieux détecter des faibles variations de température.

La présence du premier thermocouple TC1 dans le tube fin 8, crée une perte de charge répartie sur une distance bien définie, qui dépend de l'espace annulaire restant autour de la sonde. On choisit la section du tube fin 8, en fonction du diamètre du thermocouple TC1, de façon à obtenir une perte de charge importante avec des débits raisonnables compte-tenu de la disponibilité en gaz. La mesure de la variation de température du fluide corrélative à la perte de charge provoquée par le thermocouple TC1, est effectuée à l'extrémité 22 de celui-ci.

Les mesures s'effectuent en régime permanent. L'unité 14 de conditionnement en température ayant atteint une température de fonctionnement bien stable, on y connecte un moyen R d'injection de fluide sous pression tel qu'une bouteille de gaz. Après réchauffage par l'échangeur de température 16, le fluide sous pression pénètre dans le canal axial 7 de l'embout 2A et le tube fin 8. Sa température, selon les cas augmentée ou abaissée par rapport à celle qui règne dans l'unité de conditionnement 14, est mesurée par le premier thermocouple TC1 à son extrémité 22. Les signaux de mesure des deux thermocouples TC1, TC2 ainsi que les mesures des pressions amont et aval faites par les capteurs de pression PS1, PS2, sont appliquées à un calculateur 23 qui en déduit le coefficient de Joule-Thomson.

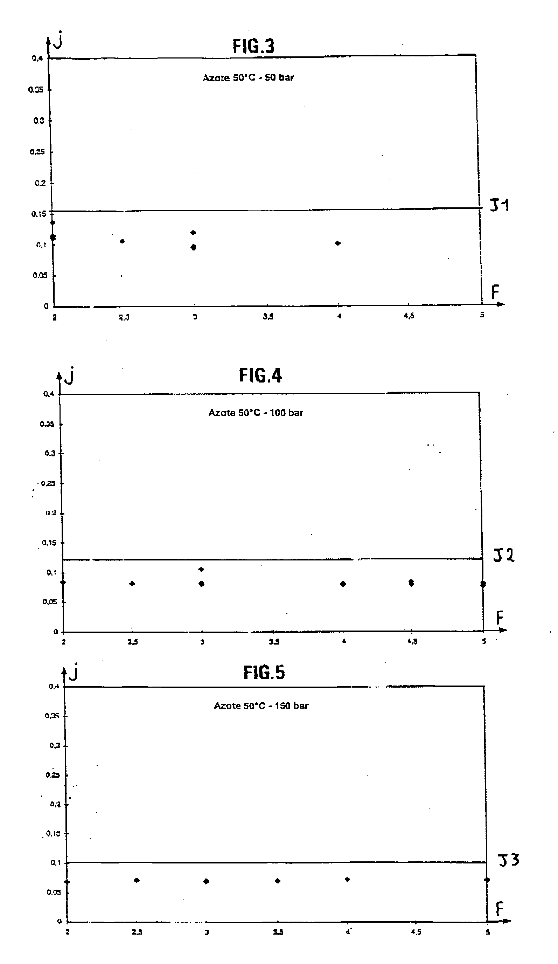

Des essais ont été menés pour valider les mesures fournies par le dispositif selon l'invention. On a injecté de l'azote successivement à différentes pressions réglées très précisément (au moyen d'un détendeur non représenté en sortie de la bouteille de gaz R) : 5Mpa, 10 Mpa et 15 Mpa. A chacune de ces pressions stabilisées, plusieurs mesures du coefficient de Joule-Thomson ont été réalisées en modifiant précisément le débit au moyen de la vanne 20. A titre de comparaison, on a déterminé les valeurs théoriques correspondantes J1, J2, J3 de ce même coefficient à partir d'une équation d'état spécifique de ce gaz pour les mêmes pressions. Comme le font apparaître les Fig.3 à 5, les résultats obtenus avec le dispositif selon l'invention, dans tous les cas expérimentaux, sont tout à fait conformes aux valeurs théoriques. Ils sont bien indépendants du débit de fluide et donc de son énergie cinétique, comme l'utilisation de la vanne 20 permet de le mettre en évidence.

## Revendications

1. Méthode pour la détermination du coefficient de Joule-Thomson d'un fluide, par combinaison de mesures des variations concomitantes de pression et de température subies par un fluide en circulation, dans laquelle on injecte le fluide à une température d'injection déterminée dans un tube fin (8), **caractérisée en ce que** l'on provoque une perte de charge pour le fluide en plaçant une sonde de température (TC1) dans ledit tube fin, on mesure, au moyen de cette sonde de température, la variation dé la température du fluide par rapport à la température d'injection, on mesure la perte de charge subie par le fluide et l'on calcule le coefficient de Joule-Thomson par combinaison des mesures de perte de charge et de variation de température du fluide.

2. Méthode selon la revendication 1, **caractérisée en ce que** l'on confine thermiquement le tube fin (8) où a lieu la mesure de la température.

3. Dispositif pour la détermination du coefficient de Joule-Thomson d'un fluide sous pression par combinaison de mesures des variations concomitantes de pression et de température subies par un fluide en circulation, comportant un tube fin (8), des moyens (R) d'injection du fluide dans le tube fin à une température déterminée, des moyens pour provoquer une perte de charge du fluide dans ledit tube fin, une première sonde de température (TC1), des moyens de mesure de pression (PS1, PS2) en amont et en aval de ce tube fin (8) pour mesurer la perte de charge, une deuxième sonde de température (TC2) de la température du fluide injecté, et un moyen de calcul (23) pour déterminer le coefficient de Joule-Thomson du fluide, **caractérisé en ce que** ladite première sonde de température (TC1) est placée dans ledit tube fin (8) constituant ainsi lesdits moyens pour provoquer une perte de charge, de façon à mesurer avec précision une température du fluide ayant subi la perte de charge.

4. Dispositif selon la revendication 3, **caractérisé en ce qu'**il comporte des moyens de confinement pour isoler thermiquement le tube fin (8).

5. Dispositif selon la revendication 4, **caractérisé en ce que** les moyens de confinement comportent un tube de confinement (2) contenant le tube fin (8), ce tube de confinement (2) étant pourvu d'embouts (2A, 2B) à ses deux extrémités opposées délimitant avec lui une enceinte étanche, des canaux (7, 11-13) dans ces deux embouts pour mettre l'intérieur du tube fin en communication avec les moyens d'injection de fluide (R), pour mettre l'intérieur du tube de confinement (2) en communication avec des moyens (P) pour y faire le vide, et pour mettre le tube fin (8) en communication avec les dits moyens de mesure de pression (PS1, PS2).

6. Dispositif selon la revendication 5, **caractérisé en ce que** les moyens de confinement comportent en outre, un tube intermédiaire (9) entre le tube fin (8) et le tube de confinement (2).

7. Dispositif selon l'un des revendications 3 à 6 **caractérisé en ce que** au moins un tube (2, 9) contenant le tube fin (8) est revêtu intérieurement d'une couche réfléchissante pour éviter les dissipations d'énergie vers l'extérieur.

8. Dispositif selon l'une des revendications 3 à 7, **caractérisé en ce qu'**il comporte une unité (14) de conditionnement du fluide en température avant son injection.

9. Dispositif selon l'une des revendications 3 à 8, **caractérisé en ce que** les sondes de température (TC1, TC2) sont des thermocouples identiques montés en opposition.

10. Dispositif selon l'une des revendications 3 à 9, **caractérisé en ce qu'**il comporte une vanne (20) pour réguler précisément le débit du fluide sortant.

## Claims

1. A method for determining the Joule-Thomson coefficient of a fluid, by combining measurements of concomitant pressure and temperature variations undergone by a circulating fluid, wherein the fluid is injected at a predetermined injection temperature into a thin tube (8), **characterized in that** a pressure drop is caused for the fluid by placing a temperature probe (TC1) in said thin tube, the variation of the fluid temperature in relation to the injection temperature is measured by means of this temperature probe, the pressure drop undergone by the fluid is measured and the Joule-Thomson coefficient is calculated by combining the measurements of the pressure drop and temperature variation undergone by the fluid.

2. A method as claimed in claim 1, **characterized in that** thin tube (8) wherein the temperature is measured is thermally confined.

3. A device for determining the Joule-Thomson coefficient of a fluid under pressure by combining measurements of concomitant pressure and temperature variations undergone by a circulating fluid, comprising a thin tube (8), means (R) for injecting the fluid into the thin tube at a predetermined temperature, means for causing a pressure drop of the fluid in said thin tube, a first temperature probe (TC1), pressure measuring means (PS1, PS2) upstream and downstream from this thin tube (8) for measuring the pressure drop, a second temperature probe (TC2) for measuring the temperature of the fluid injected, and a computation means (23) for determining the Joule-Thomson coefficient of the fluid, **characterized in that** said first temperature probe (TC1) is arranged in said thin tube (8), thus making up said means intended to cause a pressure drop, so as to measure with precision a temperature of the fluid that has undergone the pressure drop.

4. A device as claimed in claim 3, **characterized in that** it comprises confinement means for thermal insulation of thin tube (8).

5. A device as claimed in claim 4, **characterized in that** the confinement means comprise a confinement tube (2) containing thin tube (8), this confinement tube (2) being provided with terminal parts (2A, 2B) at the two opposite ends thereof, delimiting therewith a sealed enclosure, channels (7, 11-13) in these two terminal parts for communicating the inside of the thin tube with fluid injection means (R), for communicating the inside of confinement tube (2) with means (P) for creating a vacuum therein, and for communicating thin tube (8) with said pressure measuring means (PS1, PS2).

6. A device as claimed in claim 5, **characterized in that** the confinement means also comprise an intermediate tube (9) between thin tube (8) and confinement tube (2).

7. A device as claimed in any one of claims 3 to 6, **characterized in that** at least one tube (2, 9) containing thin tube (8) is internally coated with a reflecting layer to prevent energy dissipations to the outside.

8. A device as claimed in any one of claims 3 to 7, **characterized in that** it comprises a fluid temperature conditioning unit (14) for conditioning the fluid prior to injection thereof.

9. A device as claimed in any one of claims 3 to 8, **characterized in that** temperature probes (TC1, TC2) are identical thermocouples arranged in opposition.

10. A device as claimed in any one of claims 3 to 9, **characterized in that** it comprises a valve (20) for precisely controlling the flow rate of the outgoing fluid.

**Patentansprüche**

1. Verfahren zum Bestimmen des Joule-Thomson-Koeffizienten eines Fluids durch das Kombinieren von Messungen gleichzeitiger Druck- und Temperaturvariationen, die ein zirkulierendes Fluid erfährt, wobei das Fluid bei einer bestimmten Injektionstemperatur in ein dünnes Rohr (8) injiziert wird, **dadurch gekennzeichnet, dass** ein Druckverlust in dem Fluid hervorgerufen wird, indem eine Temperatursonde (TC1) in dem dünnen Rohr angeordnet wird, indem mittels dieser Temperatursonde die Variation der Fluidtemperatur bezogen auf die Injektionstemperatur gemessen wird, indem der Druckverlust gemessen wird, dem das Fluid ausgesetzt wird, und indem der Joule-Thomson-Koeffizient durch Kombinieren der Messungen des Druckverlusts und der Variation der Fluidtemperatur berechnet wird.

2. Verfahren nach Anspruch 1, **dadurch gekennzeichnet, dass** das dünne Rohr (8), in dem das Messen der Temperatur stattfindet, thermisch eingeschlossen wird.

3. Vorrichtung zum Bestimmen des Joule-Thomson-Koeffizienten eines Fluids unter Druck durch Kombinieren von Messungen gleichzeitiger Druck- und Temperaturvariationen, die ein zirkulierendes Fluid erfährt, die ein dünnes Rohr (8), Mittel (R) zur Injektion des Fluids in das dünne Rohr bei einer bestimmten Temperatur, Mittel zum Herbeiführen eines Druckverlusts des Fluids in dem dünnen Rohr, eine erste Temperatursonde (TC1), Mittel zur Druckmessung (PS1, PS2) stromaufwärts und stromabwärts dieses dünnen Rohrs (8), um den Druckverlust zu messen, eine zweite Sonde zum Messen der Temperatur (TC2) des injizierten Fluids, und ein Mittel zum Berechnen (23) umfasst, um den Joule-Thomson-Koeffizienten des Fluids zu bestimmen, **dadurch gekennzeichnet, dass** die erste Temperatursonde (TC1) in dem dünnen Rohr (8) angeordnet ist, wodurch die Mittel zum Herbeiführen eines Druckverlusts gebildet werden, um eine Temperatur des Fluids, das den Druckverlust erfahren hat, präzise zu messen.

4. Vorrichtung nach Anspruch 3, **dadurch gekennzeichnet, dass** sie Einschlussmittel umfasst, um das dünne Rohr (8) thermisch zu isolieren.

5. Vorrichtung nach Anspruch 4, **dadurch gekennzeichnet, dass** die Einschlussmittel ein Einschlussrohr (2) umfassen, das das dünne Rohr (8) enthält, wobei dieses Einschlussrohr (2) an seinen beiden gegenüberliegenden Enden mit Endstücken (2A, 2B), die mit diesem einen dichten Raum begrenzen, Kanälen (7, 11-13) in diesen beiden Endstücken ausgestattet ist, um das Innere des dünnen Rohrs mit den Mitteln zur Injektion von Fluid (R) in Kommunikation zu bringen, um das Innere des Einschlussrohr (2) in Kommunikation mit Mitteln (P) zur Vakuumerzeugung zu bringen, und um das dünne Rohr (8) mit den Mitteln zur Druckmessung (PS1, PS2) in Kommunikation zu bringen.

6. Vorrichtung nach Anspruch 5, **dadurch gekennzeichnet, dass** die Einschlussmittel ferner ein Zwischenrohr (9) zwischen dem dünnen Rohr (8) und dem Einschlussrohr (2) umfassen.

7. Vorrichtung nach einem der Ansprüche 3 bis 6, **dadurch gekennzeichnet, dass** mindestens ein Rohr (2, 9), das das dünne Rohr (8) enthält, innen mit einer reflektierenden Schicht beschichtet ist, um Energiedissipation nach außen zu verhindern.

8. Vorrichtung nach einem der Ansprüche 3 bis 7, **dadurch gekennzeichnet, dass** sie eine Einheit (14) zur Regelung der Temperatur des Fluids vor dessen Injektion umfasst.

9. Vorrichtung nach einem der Ansprüche 3 bis 8, **dadurch gekennzeichnet, dass** die Temperatursonden (TC1, TC2) identische Thermoelemente sind, die gegenüberliegend angebracht sind.

10. Vorrichtung nach einem der Ansprüche 3 bis 9, **dadurch gekennzeichnet, dass** sie ein Ventil (20) zum exakten Regulieren der Abflussmenge des austretenden Fluids umfasst.

## FIG.1

# FIG.2

## FIG.3

Azote 50°C - 50 bar

## FIG.4

Azote 50°C - 100 bar

## FIG.5

Azote 50°C - 150 bar

FIG.6

R

TC2

15

1

7

PS2

PS1

TC1

9

7

TC1

15

20

EP 1 046 909 B1

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Kortekaas W.G. et al.** Joule-Thomson Expansion of High Pressure-High-Temperature Gas Condensates. *Fluid Phase Equilibria,* 1997, vol. 139, 207-218